# EUROPEAN PATENT APPLICATION

(11) **EP 3 785 768 A1**
(43) Date of publication of application: **03.03.2021**
(21) Application number: 18912072.8
(22) Date of filing: 18.10.2018
(51) Int. Cl.: A61P 35/04, A61P 35/00, A61K 31/5377

(54) **TARGET FOR DRUG TREATMENT OF TUMOR METASTASIS AND USE THEREOF**

(30) Priority: 27.03.2018 CN 201810259369
(71) Applicant: Peking University, Beijing 100080 (CN)
(72) Inventor: ZHANG, Yu, Beijing 100083 (CN); WANG, Wei, Beijing 100083 (CN); WANG, Yuqing, Beijing 100083 (CN)
(74) Representative: EP&C
(86) International application number: PCT/CN2018/110767
(87) International publication number: WO 2019/184306

(57) **Abstract**

Disclosed is a use of PRAK as a drug target in screening a drug for inhibiting or preventing tumor cell metastasis. Based on the discovery that PRAK can be used as a target for tumor cell metastasis, further provided is a drug capable of inhibiting or preventing tumor cell metastasis. The active ingredient of the drug can reduce the expression level of PRAK or inhibit the bioactivity of PRAK, and the inhibition or prevention of tumor cell metastasis can be achieved by regulating the cell migration and the expression and/or function of HIF1α, MMP2 and/or EMT series molecules.

## Description

### Cross-reference to related application

The present application claims the priority to the Chinese patent application No. 201810259369.2, entitled "A Target in Drug Development for Tumor Metastasis and Use thereof', filed on March 27, 2018, the disclosure of which is incorporated herein by reference in its entirety.

### Technical Field

The present invention relates to the fields of biotechnology and medicine, in particular to use of PRAK as a drug target in screening a drug for inhibiting or preventing tumor cell metastasis.

### Background Art

Malignant tumors are prone to metastasis. There are four ways of metastasis: 1) Direct invasion into adjacent tissues; 2) lymphatic metastasis: cells of the primary cancer metastasize to draining lymph nodes and distant organs such as lung, liver, bone, and brain, thereby forming secondary tumors; 3) hematogenous metastasis: cancer cells are shed from the primary tumor and carried by the blood flow to distant organs, thereby forming secondary tumors within the vessels or distant organs such as lung, liver, bone and brain; 4) passive dissemination: cancer cells are shed from the primary tumor and seed on the surface of other organs or on the peritoneum or pleura. Malignant tumor metastasis has a significant impact on disease outcome.

It is estimated that 90% of the deaths of cancer patients are due to metastasis. Therefore, many efforts have been made to study how to inhibit/prevent the metastasis of tumor cells.

P38-regulated/activated protein kinase (PRAK, also known as MK5) is a member of the mitogen-activated protein kinase (MAPK) family. PRAK, as a downstream substrate of p38 MAPK, is also a kinase per se, By catalyzing the phosphorylation of various substrates, such as HSP27, ERK3/4, 14-3-3ε, p53, FOXO3 and Rheb, PRAK participates in the regulation of a number of life processes, such as cell stress, metabolism, movement, growth, and senescence. For example, under such conditions as energy exhaustion, p38 is activated, which in turn activates PRAK. By phosphorylating Rheb, PRAK inhibits mTORC1 activity, thereby regulating cell metabolism. Regarding the role of PRAK in tumor formation, existing studies have shown that, on one hand, it inhibits the occurrence of tumors by promoting cell senescence, and on the other hand, it accelerates the development of established tumors by inducing blood vessel formation. However, its role in tumor metastasis is still unknown.

### Summary of the Invention

In order to solve the problems in the prior art, the purpose of the present invention is to provide a target for the treatment of tumor metastasis and use thereof.

In order to achieve the purpose of the present invention, the technical solutions of the present invention are as follows:
The present invention first provides use of PRAK as a drug target in screening drugs for inhibiting or preventing tumor cell metastasis.

Further, the present invention provides use of PRAK inhibitors in preparing drugs for inhibiting or preventing tumor cell metastasis.

The performance of the inhibitor is to reduce the expression level of PRAK or inhibit the biological activity of PRAK. The inhibitor includes, but is not limited to, an inhibitor that specifically or non-specifically reduces the expression level of PRAK or inactivates the biological activity of PRAK. That is, as long as drugs could reduce the expression of PRAK or could reduce the biological activity of PRAK to achieve the inhibition or prevention of tumor cell metastasis, they all fall within the protection scope of the present invention.

Optionally, the inhibitor may be selected from a chemical drug, a biological macromolecule, a polypeptide, a single-chain antibody, an antisense oligonucleotide, a short hairpin RNA, a small interfering RNA, and a gene editing system.

The chemical drug includes a compound or a pharmaceutically acceptable salt thereof that can reduce the expression level of PRAK or inhibit the biological activity of PRAK

During the research of the present invention, a compound of [1,2,4] triazolo[1,5-a]pyrazine, or pharmaceutically acceptable salts, solvates, prodrugs, stereoisomers, isotopic variants and tautomers thereof for use in the treatment of degenerative and inflammatory diseases (described in the patent application with publication number CN101454326A), and a compound of imidazo[1,2-a]pyrazine, or pharmaceutically acceptable salts, solvates, prodrugs, stereoisomers, isotopic variants and tautomers thereof for use in the treatment of degenerative and inflammatory diseases (described in the patent application with publication number CN102036997A) have been found in the prior art, and they can be used as inhibitors of PRAK to inhibit the biological activity of PRAK, reduce the migration ability of tumor cells, and regulate the expression and function of HIF 1α, MMP2 and EMT-related molecules to achieve the inhibition or prevention of tumor cell metastasis. That is, the present invention provides a new use of the compound and pharmaceutical composition thereof for the inhibition or prevention of tumor cell metastasis.

Further, in the present invention, the mechanism was explored through a large number of objective tests, and it was found that the inhibitor achieves inhibition or prevention of tumor cell metastasis by inhibiting the biological activity of PRAK to reduce the migration ability of tumor cells and to regulate the expression and/or function of HIF 1α, MMP2 and/or EMT-related molecules.

Furthermore, by inhibiting the biological activity of PRAK, the inhibitor regulates the protein translation-promoting activity of mTOR to reduce the expression of HIF 1α protein.

Since PRAK is expressed in a variety of tumor cells, and HIF 1α protein is the main regulator of tumor metastasis, the tumor cells of the present invention include but are not limited to melanoma cells, breast cancer cells and the like. According to the exemplary description given in the specific embodiments of the present invention, a person skilled in the art could deduce, based on conventional knowledge, that the tumor may also include brain tumor, lung cancer, bladder cancer, gastric cancer, ovarian cancer, peritoneal cancer, pancreatic cancer, head and neck cancer, cervical cancer, endometrial cancer, colorectal cancer, liver cancer, kidney cancer, esophageal cancer, gallbladder cancer, non-Hodgkin's lymphoma, prostate cancer, thyroid cancer, female reproductive tract cancer, lymphoma, bone cancer, skin cancer, colon cancer, testicular cancer and the like.

Therefore, based on the above research results, the present invention provides a drug that could inhibit or prevent tumor cell metastasis, and the active ingredient of the drug can inhibit the biological activity of PRAK

Further, based on the above mechanism, the present invention also provides a drug that reduces migration ability of tumor cell, regulates the expression and/or function of HIF 1α, MMP2, and/or EMT-related molecules, and a drug that regulates mTOR activity. The active ingredients of the drugs can inhibit the biological activity of PRAK

It should be noted that the present invention has discovered the relationship of the biological activity of PRAK with the "mTOR activity" and the "expression and/or function of HIF 1α, MMP2 and/or EMT-related molecules". Therefore, the derivative use that is realized by using inhibitors targeting PRAK as a target to regulate the activity of mTOR, or to regulate the expression and/or function of HIF 1α, MMP2 and/or EMT-related molecules, also belongs to the protection scope of the present invention.

The experimental methods used in the present invention include (1) small molecule compound inhibitors; (2) gene-level intervention, specifically including: PRAK gene knockout mice (PRAK knockout); B16 (mouse melanoma) cell line in which PRAK is knocked out by using CRISPR-Cas9 (PRAK knockout); A375 (human melanoma)/MDA-MB-231 (human breast cancer cell line) cell lines in which PRAK is knock down by transfection with shRNA.

After preparing PRAK inhibitors, PRAK knockdown mice, and various knockout/knockdown cell lines, they are used to interrogate the implications of PRAK in the biological behaviors of melanoma and breast cancer cell lines in vitro and in tumorigenesis in mice bearing implanted tumors or sponstaneously developed breast cancer. The experimental results are as follows:
(1) Experiment in vitro:
   The results of transwell migration assay and the results of wound healing assay with B16 (mouse melanoma), A375 (human melanoma) and MDA-MB-231 (human breast cancer cell line) demonstrate that gene deletion or suppressed expression of PRAK greatly reduces the migration ability of tumor cells in vitro. On the other hand, the same procedure has no significant impact on tumor cell proliferation (MTS test) and apoptosis (annexin V-7-AAD staining).
(2) Experiment in vivo:
   A) The results of the B16 subcutaneous (s.c.) injection model show that PRAK knockout/knockdown or the use of inhibitors does not affect the growth of tumor cells at the injection site, and the tumor growth curve and final tumor size/weight are not significantly different from the wild-type group or the inhibitor-free group.
   B) The results of the B16 tail vein (i.v.) injection model show that PRAK knockout/knockdown or the use of inhibitors greatly reduces the incidence of lung metastases. The effect of inhibitors is dose-dependent. Moreover, this intervention is most effective in the early stage of tumor cell metastasis. Use of PRAK inhibitors in the first 5 days after tumor cell injection can achieve the inhibition of the distant metastasis of tumor cells, and the effect thereof is almost equal to that of the whole-course injection of inhibitors. If the inhibitor is used after this time window is missed, it is almost ineffective. ShPRAK and PRAK inhibitors have similar effects in A375 human melanoma.
   C) MDA-MB-231 in vivo imaging model: Results similar to that with the B16 tail vein injection model are obtained using the method of in vivo fluorescence detection of luciferase-expressing MDA-MB-231 cells. That is, the use of shPRAK / PRAK inhibitors strongly inhibits the colonization of tumor cells in lung. At day 14 and day 21 (the time point when the results are last acquired before the mice are sacrificed) after injection with the tumor cells, the lung fluorescence intensity of the inhibitor-treated group is much lower than that of the inhibitor-free group.
   D) Spontaneous breast cancer model in MMTV-PyMT mice: MMTV-PyMT spontaneous breast cancer mice (commercially available, for example, from Jackson Lab (JAX), 100% mice develop breast tumor from 8 to 12 weeks after birth) are crossed with PRAK knockout mice to obtain wild-type and PRAK knockout mice in the background of MMTV-PyMT. The results show that PRAK knockout greatly reduces the spontaneous lung metastasis rate in PyMT mice. Only one (1/19) of PRAK knockout PyMT mice are found to have a single metastasis in the lung. Therefore, PRAK knockout has a pronounced inhibitory effect on tumor metastasis. In contrast, the incidence and growth of tumor in the breast is not altered in the absence of PRAK. Moreover, when the tumor cells are harvested from the tumor in the breast through grounding and digestion, they show biological characteristics similar to that of tumor cell lines cultured in vitro as described above. That is, the deletion of PRAK only affects distal metastasis of tumor cells, but does not affect proliferation and apoptosis thereof. The use of PRAK inhibitors also has a similar inhibitory effect on metastasis.
(3) Potential mechanism by which PRAK regulates tumor metastasis:
   A) RNA-seq data analysis demonstrate that PRAK expression is closely related to hypoxia- and redox-related pathways;
   B) PRAK can regulate the expression and function of HIF1α, MMP2 and EMT-related molecules;
   C) PRAK can regulate the synthesis of HIF1α protein by regulating the activity of mTOR.
(4) The correlation between PRAK expression and metastasis in patents with lung adenocarcinoma/squamous cell carcinoma: comparing the primary tumor specimens from patients with tumor metastasis and those without tumor metastasis, patients with tumor metastasis expressed higher levels of PRAK (p <0.005). The expression of PRAK is positively correlated with MMP2 (*r (Spearman)=0.5050238 (p=3.651e-05)*)*.*
(5) Data mining of public databases shows that the expression of PRAK mRNA is negatively correlated with the survival rate of lung cancer patients.

On the basis of conforming to the common knowledge in the field, the above-mentioned preferred conditions could be combined with each other to obtain specific embodiment.

The beneficial effects of the present invention include:
The present invention has provided a target called PRAK, which could be used for effective inhibition of tumor metastasis, and explored the mechanism of action thereof. It is found that the inhibition or prevention of tumor cell metastasis could be achieved by suppressing the enzyme activity of PRAK or the expression level of PRAK to reduce the migration ability of tumor cells and to regulate the mTOR activity and the expression and/or function of HIF1α, MMP2 and/or EMT-related molecules.

Furthermore, the present invention has revealed the distinct characteristics of PRAK inhibitors in comparison to existing tumor chemotherapy drugs through experimental studies. Chemotherapy drugs usually exert anti-tumor effects by affecting the growth and survival of tumor cells, and their toxicity to normal tissues and drug resistance are almost inevitable. The knockout/knockdown of PRAK or the use of inhibitors does not affect the growth and survival of primary tumor cells, but effectively inhibits tumor spreading to distant organs by disrupting the metastasis process. This intervention is of greater significance in the early stage of tumor cell metastasis. More specifically, administration of PRAK inhibitors in the first 5 days after intravenous injection of tumor cells strongly inhibits the lung colonization of tumor cells, thereby preventing the formation of metastatic lesions.

### Brief Description of the Drawings

Figure 1 shows the effects of PRAK knockout and the PRAK inhibitor on the proliferation of B16-F10 cells.
Figure 2 shows the effects of PRAK knockout and the PRAK inhibitor on the apoptosis of B16-F10 cells.
Figure 3 shows the effects of PRAK knockout and the PRAK inhibitor on the migration of B16-F10 cells.
Figure 4 shows the effects of PRAK knockout and the PRAK inhibitor on the invasion of B16-F10 cells.
Figure 5 shows the effects of PRAK knockout and the PRAK inhibitor on the growth of B16-F10 tumor at the inoculation site.
Figure 6 shows the effects of PRAK knockout and the PRAK inhibitor on lung colonization of B16-F10 cells.
Figure 7 shows the impact of three different PRAK inhibitors administrated at different times on distant metastasis of B16-F10 tumor cells.
Figure 8 shows the effects of PRAK knockdown and the PRAK inhibitor on the invasion of A375 cells.
Figure 9 shows the effects of PRAK knockdown and the PRAK inhibitor on lung colonization of A375 cells.
Figure 10 shows the effects of PRAK knockdown and the PRAK inhibitor on the invasion of MDA-MB-231 cells.
Figure 11 shows the effects of PRAK knockdown and PRAK inhibitors on lung colonization of MDA-MB-231 cells.
Figure 12 shows the effects of PRAK knockout and the PRAK inhibitor on the occurrence of spontaneous breast tumors in MMTV-PyMT mice.
Figure 13 shows the effects of PRAK knockout and the PRAK inhibitor on the lung metastasis of spontaneously arising breast tumors in MMTV-PyMT mice.
Figure 14 shows the effect of PRAK knockout on the proliferation of breast tumor cells isolated from MMTV-PyMT mice.
Figure 15 shows the effect of PRAK knockout on the apoptosis of breast tumor cells isolated from MMTV-PyMT mouse.
Figure 16 shows the clustering of differentially expressed genes between wild-type and PRAK knockout B16-F10 cells as revealed by RNA Seq.
Figure 17 shows the GO pathway enrichment analysis of genes differentially expressed between wild-type and PRAK knockout B16-F10 cells.
Figure 18 shows that PRAK knockout and the PRAK inhibitor significantly reduce the protein expression of HIF-1α and MMP2 in the B16-F10 cell line.
Figure 19 shows the effect of PRAK knockout on the expression of EMT-related molecules in the B16-F10 cell line.
Figure 20 shows the altered phosphorylation of relevant proteins in the mTOR pathway induced by PRAK knockout and the PRAK inhibitor.
Figure 21 shows the detection of mRNA levels of PRAK in primary tumor samples of lung cancer patients without and with distant metastasis.
Figure 22 shows the correlation of mRNA expression levels of PRAK and MMP2 in tumor samples from lung cancer patients.
Figure 23 shows the survival analysis of lung cancer patients from the GEPIA database, grouped according to the relative expression of PRAK

### Specific Modes for Carrying Out the Embodiments

The preferred embodiments of the present invention will be described in detail below in combination with Examples. It should be understood that the following Examples are given for illustrative purposes only, and are not intended to limit the scope of the present invention. A person skilled in the art can make various changes and modifications to the present invention without departing from the purpose and spirit of the present invention.

The experimental methods used in the following Examples are conventional methods unless otherwise specified.

The materials and reagents used in the following Examples are commercially available unless otherwise specified.

The PRAK inhibitors used in the following Examples of the present invention are selected from the following compounds:

| NO. | Structures |
|---|---|
| PRAK inhibitor-23 | 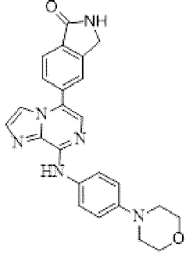 |
| **5-(8-((4-morpholinylphenyl)amino)imidazo [1,2-a]]pyrazin-5-yl)isoindol-1-one** | |
| PRAK inhibitor-22 | 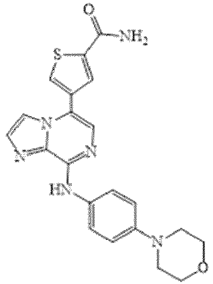 |
| **4-(8-((4-morpholinylphenyl)amino)imidazo [1,2-a]pyrazin-5-yl)thiophene-2-amide** | |
| PRAK inhibitor-29 | 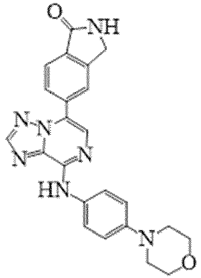 |
| **5-(8-((4-morpholinylphenyl)amino)-[1,2,4]triazolo[1,5-a] pyrazin-5-yl)isoindol-1-one** | |

### Example 1

The present Example takes the mouse melanoma cell line B16-F10 as an example to illustrate the effects of PRAK on cell proliferation survival and invasion in vitro, tumor growth upon subcutaneous inoculation and early colonization in the lung after intravenous injection.

### I. Experimental methods:

### 1. Effects on proliferation and survival in vitro

Experimental procedure:
MTS: Same numbers of PRAK WT and KO cells were plated on 96-well plates. Cell Titer 96 Aqueous cell proliferation detection solution was added at different time points after the cells adhered to the walls. After incubation at 37°C for 1 to 4 hours, the absorbance was measured at 490nm and the cell proliferation curve was drawn.
AnnexinV/7-AAD staining: Cells were plated on 24-well plates. 0.1µM and µM of PRAK inhibitor-23 were added for treatment after the cells adhered to the walls. After 24 hours, the cells were harvested for Annexin V/7-AAD staining, and the cell apoptosis was analyzed by flow cytometry.

### 2. Effects on cell migration and invasion

Experimental procedure:
Wound healing assay: Same numbers of PRAK WT and KO cells were plated on 12-well plates. After the cells adhered to the wall, the center of the well was scratched with a pipette tip. After further incubation in the medium with 0.1% FBS for 24 hours, wound healing was assessed. Similarly, the effects of different concentrations of PRAK inhibitor-23 on wound healing could be compared.
Invasion assay: The Matrigel-coated transwell chambers were pre-rehydrated at 37°C for 2 hours. 0.5×10⁵ to 2×10⁵ cells were resuspended in serum-free medium and placed in the upper chamber with the addition of 10% FBS medium to the lower chamber. After 12 to 20 hours incubation, the upper chamber was taken out and placed in pre-cooled methanol for fixation at 4°C for 15 minutes. The surface of the membrane inside the chamber was then wipe dried with a cotton swab. Crystal violet staining was performed for 20 minutes in darkness. After scraping off noninvaded cells on the top of the transwell with a cotton swab, invaded cells were counted under a light microscope.

### 3. Effects on tumor growth in situ

Experimental procedure: The C57BL/6 female mice of 6 to 8 week old (commercially available, for example, from Charles River) were selected for the experiment. The B16-F10 cells in the logarithmic growth phase were digested, and washed twice with PBS. After counting, the cells were resuspended in PBS at a density of 1.5×10⁶/ml. The tumor cells (3×10⁵ cells in 200 µL) were subcutaneously inoculated into the lateral ribs of mice. The drug-treated group was intraperitoneally injected with PRAK inhibitor-23 at 2 mg/kg/d.

The long diameter (L) and short diameter (S) of the tumor were measured with vernier calipers every two days, the tumor size was calculated with the formula L×S×S×0.5, and the tumor growth curve was drawn.

### 4. Effects on lung metastasis

Experimental procedure: The C57BL/6 female mice of 6 to 8 weeks old were selected for the experiment. The B16-F10 cells in the logarithmic growth phase were digested, and washed twice with PBS. After counting, the cells were resuspended in PBS at a density of 5×10⁵/ml. The tumor cells (1×10⁵ cells in 200 µL) were injected into mice through tail vein injection. After 15 days, the mice were sacrificed, the lungs were taken out, and the number of tumor nodules in the lung surface was counted. The drug-treated group was administrated on days 0 to 4 or days 5 to 15, respectively by intraperitoneal injection of PRAK inhibitor-23 at 2mg/kg/d.

### II. Experimental results:

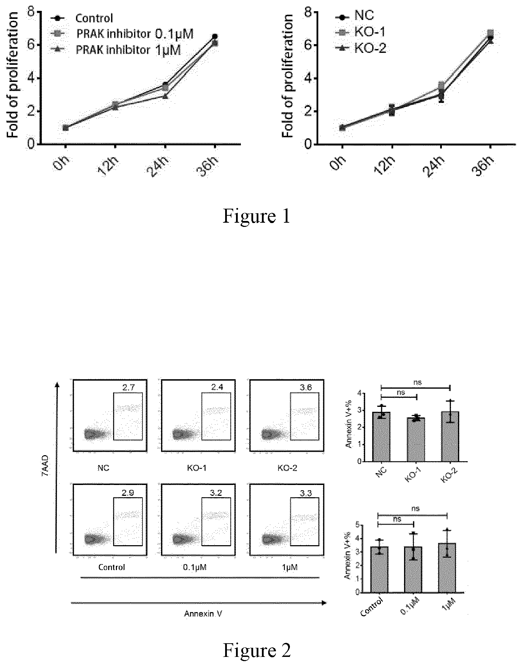

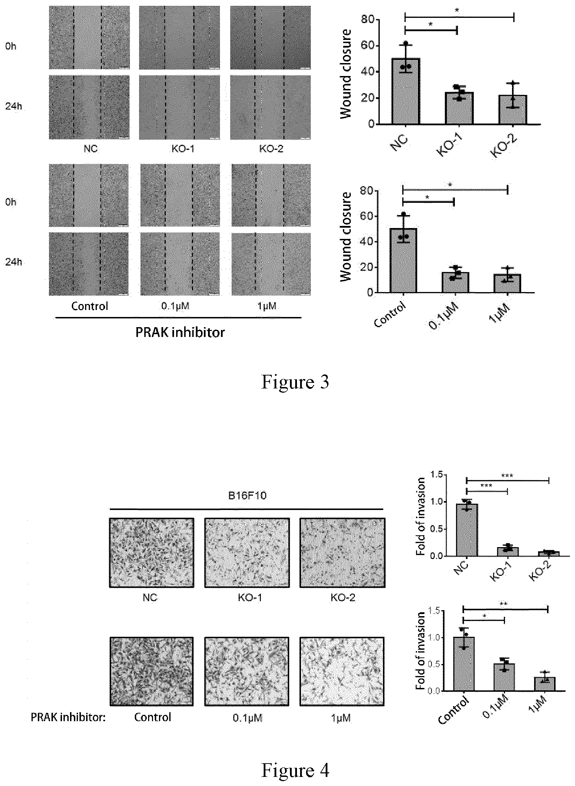

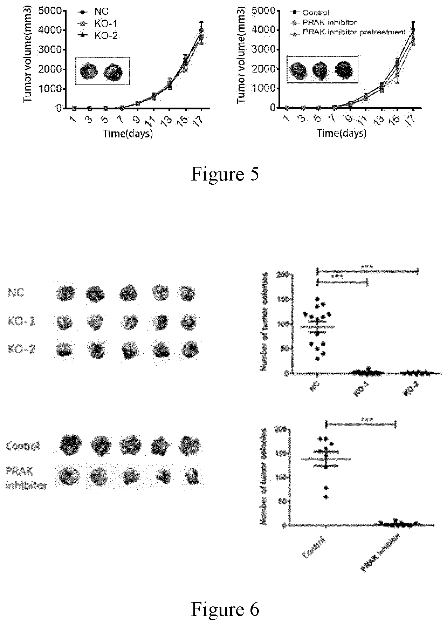

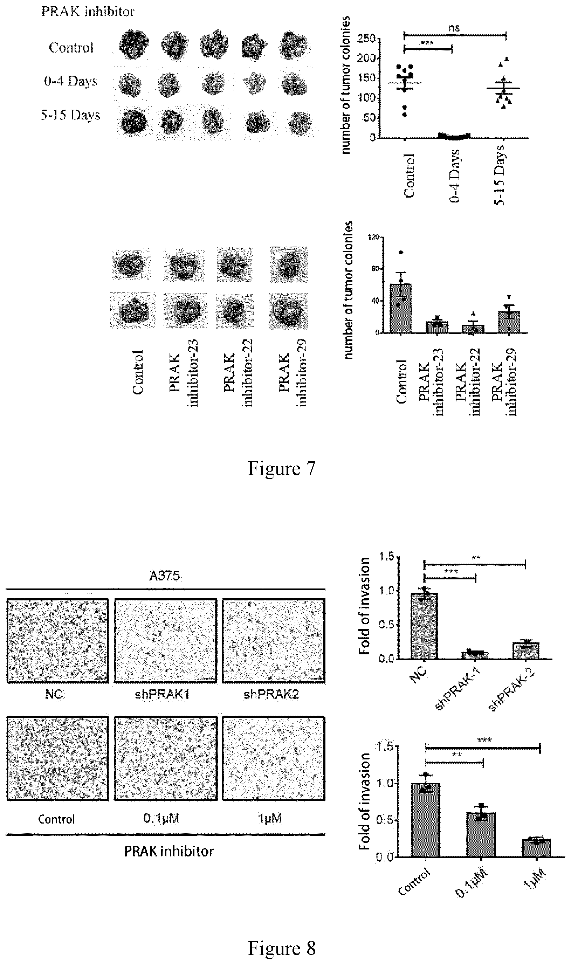

1. As shown in Figure 1, PRAK knockout and PRAK inhibitor has no significant effect on the proliferation of B16-F10 cells.
2. As shown in Figure 2, PRAK knockout and PRAK inhibitor has no significant effect on the apoptosis of B16-F10 cells.
3. As shown in Figure 3, PRAK knockout and PRAK inhibitor could significantly inhibit the migration of B16-F10 cells.
4. As shown in Figure 4, PRAK knockout and PRAK inhibitor could significantly inhibit the invasion of B16-F10 cells.
5. As shown in Figure 5, PRAK knockout and PRAK inhibitor have no significant effect on the growth of subcutaneously inoculated B16-F10 tumors at the injection site.
6. As shown in Figure 6, PRAK knockout and PRAK inhibitor could significantly inhibit the ability of intravenously injected B16-F10 cells to colonize in the lung.
7. As shown in Figure 7, the use of PRAK inhibitors on days 0 to 4 after intravenous injection of tumor cells could significantly inhibit the lung metastasis of B16-F10 tumor cells. The use of PRAK inhibitors on days 5 to 15 has no obvious inhibitory effect. Not only that, the experimental results also show that the three different PRAK inhibitors have similar inhibitory effects.

It is confirmed that PRAK knockout and PRAK inhibitor have no significant effect on the proliferation and growth of B16-F10 cells in vitro or at the site of subcutaneous injection, but can significantly inhibit the invasion capacity and the ability of B16-F10 to colonize in the lung.

### Example 2

The present Example takes the human melanoma cell line A375 as an example to illustrate the effects of PRAK on the invasion and lung colonization ability of tumor cells.

### I. Experimental methods:

The experiment procedure was performed as in Example 1, except that B16-F10 was replaced with A375, PRAK knockdown was conducted by transfection with shRNA, and the recipient mouse for tumor inoculation was SCID-Beige (commercially available, for example, from Charles River).

### II. Experimental results:

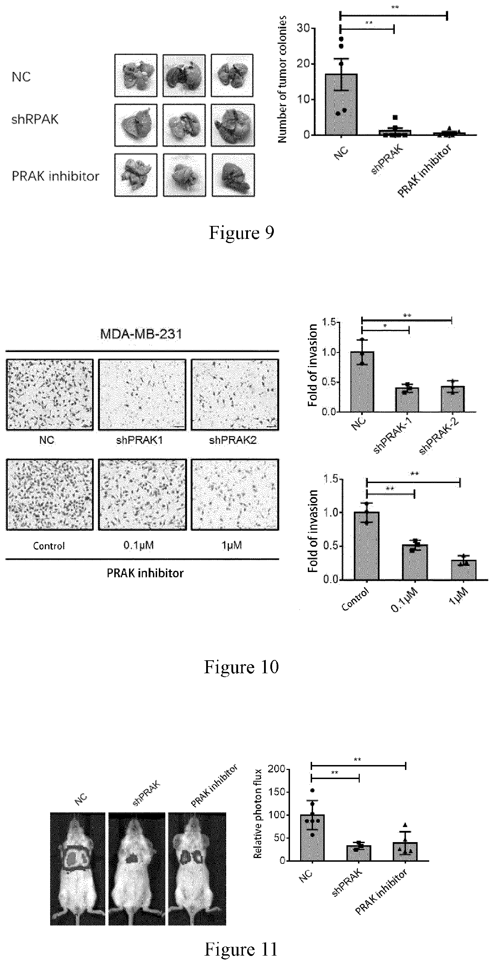

1. As shown in Figure 8, PRAK knockdown and PRAK inhibitor could significantly inhibit the invasion ability of A375.
2. As shown in Figure 9, PRAK knockdown and PRAK inhibitor could significantly inhibit the ability of A375 to colonize in lung.

It is confirmed that PRAK knockdown and PRAK inhibitor could significantly inhibit the invasion and lung colonization ability of the human melanoma cell A375.

### Example 3

The present Example takes a human breast cancer cell line as an example to illustrate the effect of PRAK on the invasion and lung colonization ability of MDA-MB-231 cells.

### I. Experimental methods:

Experimental procedure: The SCID-Beige female mice of 6 to 8 weeks old were selected for the experiment. The wild-type or PRAK shRNA transfected luciferase-expressing MDA-MB-231 cells in logarithmic growth phase were digested, and washed twice with PBS. After counting, the cells were resuspended in PBS at a density of 2.5×10⁶/ml. The tumor cells (5×10⁵ cells in 200 µL) were injected into mice via tail vein injection. The growth of tumor cells in the lungs of recipient mice was monitored by bioluminescence imaging using the IVIS Spectrum in vivo imaging system for small animals. The drug-treated group was intraperitoneally injected with PRAK inhibitor at 2 mg/kg/d in the first five days after tumor injection.

### II. Experimental results:

1. As shown in Figure 10, PRAK knockdown and PRAK inhibitor could significantly inhibit the invasion ability of MDA-MB-231 cells.
2. As shown in Figure 11, PRAK knockdown and PRAK inhibitor could significantly inhibit the ability of MDA-MB-231 to colonize in the lung.

It is confirmed that PRAK knockdown and PRAK inhibitor can significantly inhibit the invasion and lung colonization ability of human breast cancer cell MDA-MB-231.

### Example 4

The present Example takes MMTV-PyMT mice with spontaneous breast cancer as an example to illustrate the effect of PRAK on lung metastasis of spontaneously arising breast cancer.

### I. Experimental methods:

### 1. The effect on the occurrence and growth of primary breast tumors in mice

The MMTV-PyMT mice were crossed with PRAK mice to obtain MMTV-PRAK WT and MMTV-PRAK knockout mice. From 8 to 10 weeks, the occurrence of breast tumors was observed. Another group of MMTV-PRAK WT mice were given PRAK inhibitor (1mg/kg) every other day from the 12th week. At week 15, the mice were sacrificed, breast tumor nodules were counted and weighed, and lung tumor nodules were counted at the same time.

### 2. Effect on the proliferation and apoptosis of primary mouse tumor cells in vitro

Experimental procedure: The primary mammary tumors was taken out, cut into pieces, and single cell suspension of tumor cells were prepared through digestion with DNase and collagenase and density gradient centrifugation, and subjected to MTS analysis and AnnexinV/7-AAD staining.

### II. Experimental results:

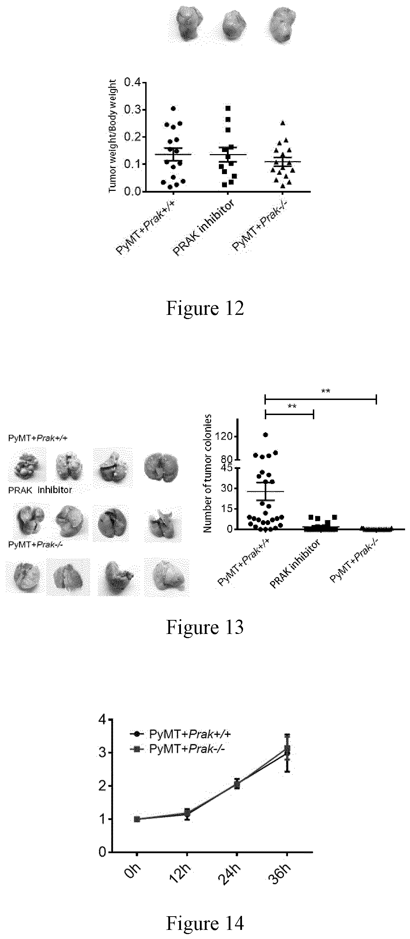

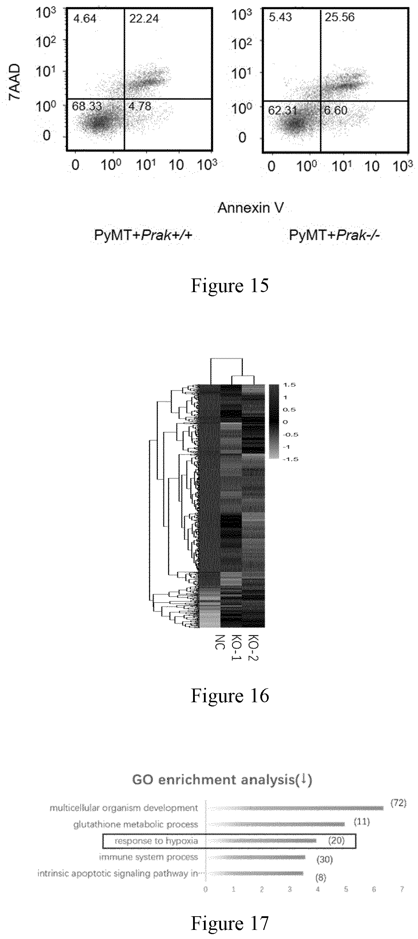

1. As shown in Figure 12, PRAK knockout and PRAK inhibitor have no significant effect on the incidence and growth of spontaneous breast tumors.
2. As shown in Figure 13, PRAK knockout and PRAK inhibitor significantly inhibited the lung metastasis of spontaneously arising breast tumors in mice.
3. As shown in Figure 14, PRAK knockout has no significant effect on the proliferation of tumor cells isolated from spontaneous breast tumors in mice.
4. As shown in Figure 15, PRAK knockout has no significant effect on the apoptosis of tumor cells isolated from spontaneous breast tumors in mice.

### Example 5

The present Example is used to illustrate the effect of PRAK knockout on the gene transcription profile of the B16-F10 cell line.

### I. Experimental method:

### 1. RNA was extracted from PRAK WT and PRAK knockout B16-F10 cells. The transcriptional profiles were determined using RNA Seq. The differentially expressed genes were subjected to GO enrichment analysis.

### II. Experimental results:

1. As shown in Figure 16, compared with the wild type, most of the differentially expressed genes after PRAK gene knockout are down-regulated, whereas a small number of genes are up-regulated.
2. As shown in Figure 17, the functional enrichment analysis of differentially expressed genes between PRAK WT and PRAK knockout shows that many genes down-regulated after PRAK knockout are related to hypoxia.

### Example 6

The present Example is used to illustrate the effect of PRAK on the expression and function of HIF1α, MMP2 and/or EMT-related molecules.

### I. Experimental method:

The changes in protein levels of individual molecules after PRAK knockout and PRAK inhibitor treatment was detected by Western Blot analysis.

### II. Experimental results:

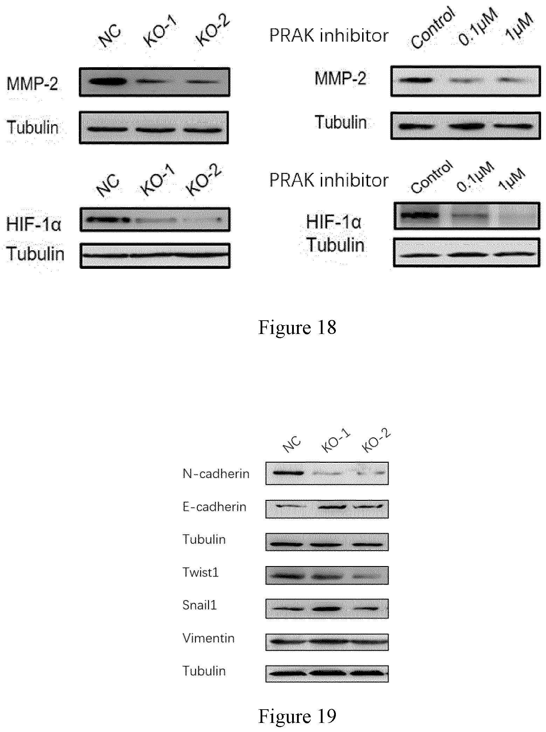

1. As shown in Figure 18, PRAK knockout and PRAK inhibitor treatment could significantly reduce the expression levels of HIF-1α and MMP2 in the B16-F10 cell line.
2. As shown in Figure 19, PRAK knockout could significantly reduce the expression of N-cadherin but increase the expression of E-cadherin in the B16-F10 cell line.

### Example 7

The present Example is used to illustrate the effect of PRAK on the expression of mTOR-related molecules.

### I. Experimental methods:

The changes in the protein phosphorylation level of individual molecules after PRAK knockout and PRAK inhibitor treatment was detected by Western Blot analysis.

### II. Experimental results:

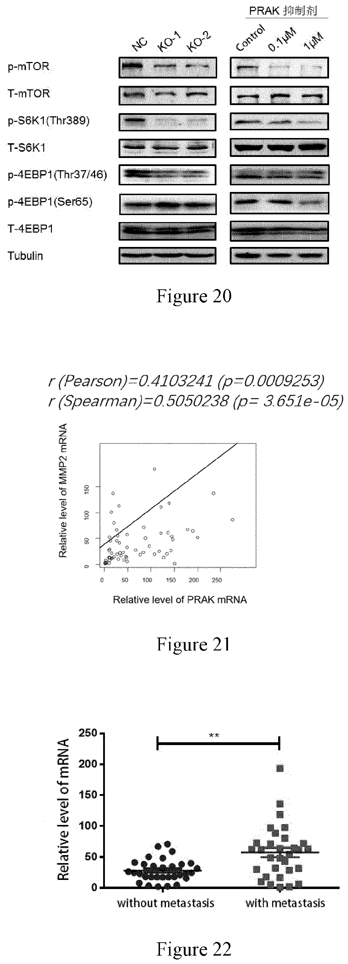

As shown in Figure 20, after PRAK knockout and PRAK inhibitor treatment, the phosphorylation level of mTOR pathway-related molecules was reduced.

### Example 8

The present Example is used to illustrate the correlation between PRAK expression and the tumor metastasis of lung cancer patients.

### I. Experimental methods:

1. Tumor specimens from patients with lung adenocarcinoma and lung squamous cell carcinoma were collected, and mRNA level of PRAK expression was detected. The patients were divided into a group without distant metastasis and a group with distant metastasis based on the postoperative follow-up. The results were subjected to statistical analysis.
2. PRAK and MMP2 mRNA expression was determined in the above patient specimens, and the results were subjected to correlation analysis.
3. Effect of PRAK expression on the survival of lung cancer patients was analyzed using GEPIA database.

### II. Experimental results:

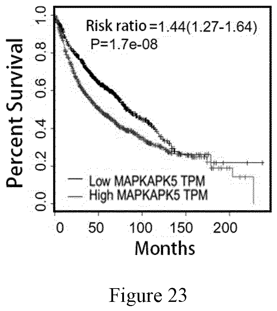

1. As shown in Figure 21, compared to patients without distant metastases, patients with metastases have higher expression of PRAK in their tumor specimens.
2. As shown in Figure 22, the expression levels of PRAK and MMP2 in tumor patient specimens are positively correlated.
3. As shown in Figure 23, the overall survival of lung cancer patients is significantly correlated with PRAK expression. Patients with higher expression of PRAK have a shorter survival time than patients with lower expression of PRAK

General Explanation: PRAK inhibitor-23 was used in Examples 2 through 9. The same experiments were also performed using PRAK inhibitor-22 or PRAK inhibitor-29 with similar results. The experiments verified that the three inhibitors share similar functions (all can achieve the same inhibitory effect).

Although the general description and specific embodiments have been used to describe the present invention in detail above, some modifications or improvements can be made on the basis of the present invention, which is obvious to a person skilled in the art. Therefore, these modifications or improvements made without departing from the spirit of the present invention belong to the scope of the present invention.

### Industrial applicability

The present invention discloses use of PRAK as a drug target in screening a drug for inhibiting or preventing tumor cell metastasis. Based on the discovery that PRAK can be used as a target for tumor cell metastasis, the present invention further provides a drug capable of inhibiting or preventing tumor cell metastasis. The active ingredient of the drug can reduce the expression level of PRAK or inhibit the biological activity of PRAK, to regulate the cell migration and the expression and/or function of HIF1α, MMP2 and EMT-related molecules, thereby achieve inhibition or prevention of tumor cell metastasis. The present invention has good economic value and application prospects.

## Claims

1. Use of PRAK as a target in the screening of drugs for inhibiting or preventing tumor cell metastasis.

2. Use of a PRAK inhibitor in preparing a drug for inhibiting or preventing tumor cell metastasis.

3. The use according to claim 2, wherein the inhibitor regulates the cell migration and the expression and/or function of HIF1α, MMP2 and/or EMT-related molecules by reducing the expression level of PRAK or inhibiting the biological activity of PRAK, thereby inhibiting or preventing tumor cell metastasis.

4. The use according to claim 3, wherein the inhibitor regulates the expression of mTOR and substrates thereof and further regulates the expression of HIF1α protein, by reducing the expression level of PRAK or inhibiting the biological activity of PRAK

5. A drug for inhibiting or preventing tumor cell metastasis, wherein the active ingredient of the drug is capable of reducing the expression level of PRAK or inhibiting the biological activity of PRAK

6. The drug according to claim 5, wherein the drug is selected from a chemical drug, a biological macromolecule, a polypeptide, a single-chain antibody, an antisense oligonucleotide, a short hairpin RNA, a small interfering RNA, and a gene editing system.

7. The drug according to claim 6, wherein the drug comprises a [1,2,4] triazolo[1,5-a]pyrazine compound, or a pharmaceutically acceptable salt, a solvate, a prodrug, a stereoisomer, an isotopic variant or a tautomer thereof.

8. The drug according to claim 6, wherein the drug comprises an imidazo[1,2-a]pyrazine compound, or a pharmaceutically acceptable salt, a solvate, a prodrug, a stereoisomer, an isotopic variant or a tautomer thereof.

9. A drug for regulating the expression and/or function of HIF1α, MMP2 and/or EMT-related molecules, wherein the active ingredient of the drug is capable of reducing the expression level of PRAK or inhibiting the biological activity of PRAK

10. A drug for regulating the activity of mTOR, wherein the active ingredient of the drug is capable of reducing the expression level of PRAK or inhibiting the biological activity of PRAK
